# EUROPÄISCHE PATENTSCHRIFT

(11) **EP 1 001 756 B1**
(45) Veröffentlichungstag und Bekanntmachung des Hinweises auf die Patenterteilung: **07.04.2004**
(21) Anmeldenummer: 98929341.0
(22) Anmeldetag: 19.05.1998
(51) Int. Cl.: A61K 31/19, A61K 31/60, A61P 35/00

(54) **SYNERGISTISCH WIRKENDE ZUSAMMENSETZUNGEN ZUR SELEKTIVEN BEKÄMPFUNG VON TUMORGEWEBE**
SYNERGISTICALLY ACTING COMPOSITIONS FOR SELECTIVELY COMBATING TUMOR TISSUE
COMPOSITIONS A EFFET SYNERGIQUE POUR LUTTER SELECTIVEMENT CONTRE LES TISSUS TUMORAUX

(30) Priorität: 24.06.1997 DE 19726871
(43) Veröffentlichungstag der Anmeldung: 24.05.2000
(73) Patentinhaber: Kreutz, Werner, Prof. Dr., 79219 Staufen (DE)
(72) Erfinder: Kreutz, Werner, Prof. Dr., 79219 Staufen (DE)
(74) Vertreter: Best, Michael, Dr.
(86) Internationale Anmeldenummer: PCT/EP1998/002939
(87) Internationale Veröffentlichungsnummer: WO 1998/058639

(56) Entgegenhaltungen:
- WO-A-96/30003
- DE-A- 4 407 484
- US-A- 4 871 570
- DATABASE WPI Section Ch, Week 8801 Derwent Publications Ltd., London, GB; Class B05, AN 88-003456 XP002076797 & JP 62 267 232 A (ARAI S)

## Beschreibung

Die Erfindung betrifft neue synergistisch wirkende Zusammensetzungen, die selektiv Tumorgewebe bekämpfen, während gesundes Gewebe praktisch nicht angegriffen wird. Die neuen Zusammensetzungen eignen sich daher hervorragend für die Krebstherapie.

Arzneimittel nach dem Stand der Technik, die bei der Chemotherapie eingesetzt werden, bringen in der Regel nur Teilerfolge, d.h. sie führen zu keiner endgültigen Heilung. Darüberhinaus wirken die im Stand der Technik eingesetzten Substanzen häufig nur bei einer bestimmten Tumorkategorie. Ein weiterer Nachteil der derzeit bekannten Chemotherapeutika sind ihre oft schädlichen Nebenwirkungen, da Chemotherapeutika generell auf proliferierende Gewebe cytostatisch wirken können. Die bekannten Chemotherapeutika sind auch bei der Bekämpfung der Metastasenbildung nicht zufriedenstellend, und dies ist einer der Hauptgründe, die bislang einen entscheidenden Erfolg bei der Krebstherapie verhinderten.

Daß Tumorgewebe im extrazellulären Milieu einen abgesenkten mittleren pH-Wert von etwa 6,5 bis 7,0 aufweist und der pH-Wert auf der Krebszellenoberfläche sogar bis 5 absinken kann, während der pH-Wert im Normalgewebe und im Blut etwa 7,2 bis 7,5 beträgt, ist bekannt und wird z.B. in der DE-A 44 07 484 und in Tumor Biol., 1994, 15: 304-310 beschrieben. So weist jede Tumorart einen intrinsischen mittleren interzellulären pH-Wert auf, der z.B. bei Brusttumoren ca. 6,7 und bei Colontumoren ca. 6,9 beträgt.

In den vorstehend genannten Druckschriften wird offenbart, daß durch die Absenkung des pH-Bereichs in Tumorzellen die natürliche Immunabwehr blockiert wird, da die körpereigenen Abwehrzellen mit voller Cytotoxizität nur im leicht basischen Milieu von mehr als 7 auf Krebs-Target-Zellen reagieren. Die DE-A 44 07 484 schlägt daher vor, das saure externe Milieu von Krebszellen auf das normale physiologische pH-Niveau von 7 bis 7,5 zu bringen und dadurch die Krebszellen durch die körpereigene Immunabwehr zu bekämpfen. Hierzu wird das saure externe Milieu von Krebszellen entweder durch künstliche Alkalisierungsmaßnahmen oder durch die Verhinderung des Ansäuerungsprozesses selbst auf einen physiologischen pH-Wert von 7 bis 7,5 gebracht.

Die in der DE-A 44 07 484 beschriebenen Arzneimittel stellen zwar einen Fortschritt in der Krebstherapie dar, es wäre jedoch wünschenswert, Arzneimittel zur Verfügung zu haben, die neben der körpereigenen Immunabwehr Tumorzellen selektiv bekämpfen und damit als relativ nebenwirkungsarme Chemotherapeutika verwendet werden können.

Entsprechend schlägt die WO 96/30003 vor, solche Verbindungen zur Bekämpfung von Tumorgewebe zu verwenden, die bei einem pH-Wert von kleiner 7 protoniert werden oder eine Substanz freisetzen, wobei die protonierte Verbindung oder die freigesetzte Substanz auf Zellen stärker zerstörend wirkt als die unprotonierte Verbindung bzw. die Verbindung vor Freisetzen der Substanz. Die WO 96/30003 offenbart für diese Verbindungen allgemeine Formeln, unter die eine Vielzahl von chemischen Verbindungen fallen. Als wirkungsvoll wird dort unter anderem auch die 4-Amino-2-hydroxybenzoesäure genannt. Auch Acetylsalicylsäure wird als mögliche wirksame Verbindung genannt, jedoch ist diese Verbindung nicht bevorzugt.

Die Verbindungen der WO 96/30003 und auch die dort allgemein vorgeschlagenen Gemische aus zwei und mehr Verbindungen weisen zwar bereits eine gute Anti-Tumorwirkung auf, es besteht jedoch nach wie vor ein Bedarf an Arzneimitteln, die eine verbesserte Anti-Tumorwirkung zeigen, insbesondere bei pH-Werten von 7,0 oder darunter, insbesondere im Bereich von 6,5 bis 7,0.

Es ist daher eine Aufgabe der vorliegenden Erfindung, neue Arzneimittel zur Verfügung zu stellen, die eine starke cytotoxische Wirkung weitgehend selektiv auf Tumorgewebe aufweisen, insbesondere in einem pH-Bereich von etwa 6,5 bis etwa 7,0.

Diese Aufgabe wird durch den Gegenstand der Patentansprüche gelöst.

Die erfindungsgemäßen Zusammensetzungen wirken im Prinzip auf die gleiche Art und Weise wie die in der WO 96/30003 offenbarten Benzoesäurederivate. Es hat sich jedoch gezeigt, daß diese aus der WO 96/30003 bekannten Benzoesäurederivate nicht vollständig zufriedenstellend wirksam gegen Tumorgewebe sind. Überraschenderweise weisen aber einzelne Benzoesäurederivate im Gemisch miteinander eine sehr stark synergistische Wirkung für die Abtötung von Tumorgewebe auf und rufen in einem pH-Bereich von etwa 7,0 und darunter einen praktisch vollständigen Zelltod bei Tumorgewebe hervor.

Bei den erfindungsgemäßen Zusammensetzungen handelt es sich um Gemische, die folgende Verbindungskombinationen enthalten:
2,6-Dihydroxy- und 2-Hydroxy-4-aminobenzoesäure,
2,6-Dihydroxy- und 2-Acetoxybenzoesäure (Acetylsalicylsäure),
2,6-Dihydroxy benzoe- und Salicylsäure,
2,6-Dihydroxy- und 2,4-Diacetoxybenzoesäure,
2,6-Dihydroxy- und 2,4-Dimethoxybenzoesäure,
2-Hydroxy-4-amino benzoe- und Salicylsäure,
2-Hydroxy-4-amino- und 2-Acetoxybenzoesäure (Acetylsalicylsäure),
2-Hydroxy-4-amino- und 2,4-Dimethoxybenzoesäure,
2,4-Dimethoxy- und 2-Acetoxybenzoesäure,
2,4-Dimethoxy benzoe- und Salicylsäure,
2,4,6-Trihydroxy- und 2,4-Dimethoxybenzoesäure,
2,4,6-Trihydroxy- und 2,6-Dihydroxybenzoesäure,
2,4,6-Trimethoxy- und 2,6-Dihydroxybenzoesäure,
2,4,6-Trimethoxy- und 2-Hydroxy-4-aminobenzoesäure,
2,4,6-Trimethoxy- und 2,4,6-Trihydroxybenzoesäure,
2,4,6-Trimethoxy- und 2,4-Dimethoxybenzoesäure,
2-Hydroxy-4-aminobenzoesäure und 5-(2,4-Difluorophenyl)-salicylsäure,
2-Acetoxybenzoesäure und 5-(2,4-Difluorophenyl)salicylsäure, Salicylsäure und 5-(2,4-Difluorophenyl)salicylsäure,
2-Acetoxybenzoesäure und α-Cyano-3-hydroxyzimtsäure,
Salicylsäure und α-Cyano-3-hydroxyzimtsäure,
5-(2,4-Difluorophenyl)salicylsäure und α-Cyano-3-hydroxyzimtsäure,
2-Hydroxy-4-aminobenzoesäure und α-Cyano-3-hydroxyzimtsäure,
2-Hydroxy-4-aminobenzoesäure und 2-Acetoxybenzoesäure und 5-(2,4-Difluorophenyl)salicylsäure,
2-Hydroxy-4-aminobenzoesäure und Salicylsäure und 5-(2,4-Difluorophenyl)salicylsäure,
2-Hydroxy-4-aminobenzoesäure und 2-Acetoxybenzoesäure und α-Cyano-3-hydroxyzimtsäure,
2-Hydroxy-4-aminobenzoesäure und Salicylsäure und α-Cyano-3-hydroxyzimtsäure,
2-Hydroxy-4-aminobenzoesäure und 5-(2,4-Difluorophenyl)-salicylsäure und α-Cyano-3-hydroxyzimtsäure,
2-Acetoxybenzoesäure und 5-(2,4-Difluorophenyl)salicylsäure und α-Cyano-3-hydroxyzimtsäure oder
Salicylsäure und 5-(2,4-Difluorophenyl)salicylsäure und α-Cyano-3-hydroxyzimtsäure.

Die einzelnen Verbindungen sind als solche bekannt, im Handel erhältlich und können von einem Fachmann ohne weiteres hergestellt werden. Ihren besonderen therapeutischen Nutzen zeigen sie jedoch erst in den erfindungsgemäßen Zusammensetzungen. Die Zusammensetzungen wirken synergistisch.

Die synergistische Wirkung kann von einem Fachmann unter Berücksichtigung der nachstehenden Ausführungen leicht festgestellt werden.

Synergistisch wirkende Zusammensetzungen sind beispielsweise folgende Gemische:
2,6-Dihydroxybenzoesäure / 2-Hydroxy-4-aminobenzoesäure
2,6-Dihydroxybenzoesäure / Acetylsalicylsäure
2,6-Dihydroxybenzoesäure / 2,4-Diacetoxybenzoesäure
2,6-Dihydroxybenzoesäure / 2,4-Dimethoxybenzoesäure
2-Hydroxy-4-aminobenzoesäure / Acetylsalicylsäure
2-Hydroxy-4-aminobenzoesäure / 2,4-Dimethoxybenzoesäure und
2,4-Dimethoxybenzoesäure / 2-Acetoxybenzoesäure.

Weiterhin können auch folgende Gemische genannt werden:
2,4,6-Trihydroxybenzoesäure / 2,4-Dimethoxybenzoesäure
2,4,6-Trihydroxybenzoesäure / 2,6-Dihydroxybenzoesäure
2,4,6-Trimethoxybenzoesäure / 2,6-Dihydroxybenzoesäure
2,4,6-Trimethoxybenzoesäure / 2-Hydroxy-4-aminobenzoesäure
2,4,6-Trimethoxybenzoesäure / 2,4,6-Trihydroxybenzoesäure
2,4,6-Trimethoxybenzoesäure / 2,4-Dimethoxybenzoesäure.

Als weitere Gemische können genannt werden:
2-Hydroxy-4-aminobenzoesäure / 5-(2,4-Difluorophenyl)salicylsäure
2-Acetoxybenzoesäure / 5-(2,4-Difluorophenyl)salicylsäure
2-Acetoxybenzoesäure / α-Cyano-3-hydroxyzimtsäure
5-(2,4-Difluorophenyl)salicylsäure / α-Cyano-3-hydroxyzimtsäure und
2-Hydroxy-4-Aminobenzoesäure / α-Cyano-3-hydroxyzimtsäure.

Besonders bevorzugt werden die Gemische auch in Dreierkombinationen eingesetzt, wobei folgende Dreierkombinationen vorteilhafte synergistische Eigenschaften aufweisen:
2-Hydroxy-4-aminobenzoesäure / 2-Acetoxybenzoesäure / 5-(2,4-Difluorophenyl)salicylsäure
2-Hydroxy-4-aminobenzoesäure / 2-Acetoxybenzoesäure / α-Cyano-3-hydroxyzimtsäure
2-Hydroxy-4-aminobenzoesäure / 5-(2,4-Difluorophenyl)salicylsäure / α-Cyano-3-hydroxyzimtsäure und
2-Acetoxybenzoesäure / 5-(2,4-Difluorophenyl)salicylsäure / α-Cyano-3-hydroxyzimtsäure.

In den vorstehenden Gemischen kann anstelle der Acetylsalicylsäure auch die 2-Hydroxybenzoesäure (Salicylsäure) verwendet werden.

Die erfindungsgemäßen Zusammensetzungen werden aufgrund ihrer pH-Sensitivität nur in Krebstumoren und Metastasenbereichen aktiviert und stellen deshalb ein ideales Krebstherapeutikum dar. Auch ist besonders hervorzuheben, daß dieses neue Krebstherapeutikum unabhängig von der speziellen Krebsart generell auf alle Tumortypen wirkt.

Es wird angenommen, daß die erfindungsgemäßen Substanzgemische im protonierten Zustand toxische Eigenschaften aufweisen und auf die Tumoren als Zellgifte wirken.

Es ist bekannt, daß die pH-Werte im extrazellulären Tumorgewebe nochmals um ca. 0,5 pH-Einheiten abgesenkt werden können, indem man durch Glucoseverabreichung Acidosen induziert. Eine solche Verabreichung von Glucose kann ebenfalls mit den erfindungsgemäßen Zusammensetzungen erfolgen.

Die erfindungsgemäßen Zusammensetzungen können die Wirkstoffe in beliebigen Anteilen aufweisen, sofern der synergistische Effekt noch auftritt. Wie die Wirksamkeit von Verbindungen und damit auch das Vorliegen eines synergistischen Effekts nachgewiesen werden kann, ist z.B. in der WO 96/30003 ausführlich beschrieben, und insoweit kann auf diese Druckschrift Bezug genommen werden. Ferner wird auf die folgenden Vergleichsbeispiele verwiesen.

Bevorzugt weisen die erfindungsgemäßen Zusammensetzungen die beiden Wirkstoffe im Verhältnis 1:9 bis 9:1, besonders bevorzugt im Verhältnis 2:1 bis 1:2 und insbesondere im Verhältnis von etwa 1:1 auf.

Die synergistische Wirksamkeit der erfindungsgemäßen Zusammensetzungen wurde durch in vitro Versuche eindeutig belegt. Im folgenden werden für folgende Substanzennamen folgende Kürzel verwendet:
Substanz 121 = Acetylsalicylsäure (2-Acetoxybenzoesäure)
Substanz 58a = 2-Hydroxy-4-aminobenzoesäure
Substanz 132 = 2,4-Diacetoxybenzoesäure
Substanz 136 = 2,6-Dihydroxybenzoesäure
Substanz 188 = 2,4-Dimethoxybenzoesäure.

Die erfindungsgemäßen Versuche wurden wie folgt durchgeführt:

Die Messungen wurden mit einem bei der Firma Boehringer (Mannheim) käuflichen "Cell-Death-Detection ELISA"-Kit, Kat.-Nr. 1774 425, durchgeführt. Die Verfahrensvorschrift wird von der Firma Boehringer mitgeliefert.

Die Ergebnisse können folgenden Tabellen 1 bis 3 entnommen werden. Der OD-Wert entspricht dem Zelltod.

**Tabelle 1**

| (Photometrische Messung nach 35 Minuten) | | |
|---|---|---|
| Verbindung | initialer pH | OD (405-490 nm) |
| | pH 6,0 | 0,818 |
| CAM (800 ng/ml) | pH 6,5 | 3,037 |
| | pH 7,0 | 2,725 |
| | pH 7,4 | 2,416 |
| | pH 6,0 | 0,187 |
| 136 (5 mM) | pH 6,5 | 0,918 |
| | pH 7,0 | 0,321 |
| | pH 7,4 | 0,382 |
| | pH 6,0 | 0,221 |
| 136 (7 mM) | pH 6,5 | 1,287 |
| | pH 7,0 | 0,524 |
| | pH 7,4 | 0,816 |
| | pH 6,0 | 0,642 |
| Negativkontrolle | pH 6,5 | 0,702 |
| | pH 7,0 | 0,293 |
| | pH 7,4 | 0,268 |
| Positivkontrolle | | 3,145 |

**Tabelle 2**

| (Photometrische Messung nach 35 Minuten) | | |
|---|---|---|
| Verbindung | initialer pH | OD (405-490 nm) |
| | pH 6,0 | 0,344 |
| CAM (160 ng/ml) | pH 6,5 | 0,711 |
| | pH 7,0 | 1,220 |
| | pH 7,4 | 1,923 |
| | pH 6,0 | 0,320 |
| 58a (15 mM) | pH 6,5 | 0,282 |
| | pH 7,0 | 0,574 |
| | pH 7,4 | 1,835 |
| | pH 6,0 | 1,282 |
| 121 (10 mM) | pH 6,5 | 0,163 |
| | pH 7,0 | 0,413 |
| | pH 7,4 | 1,508 |
| | pH 6,0 | 2,856 |
| 132 (10 mM) | pH 6,5 | 0,183 |
| | pH 7,0 | 0,088 |
| | pH 7,4 | 0,502 |
| | pH 6,0 | 0,256 |
| 188 (15 mM) | pH 6,5 | 0,309 |
| | pH 7,0 | 0,502 |
| | pH 7,4 | 1,854 |
| | pH 6,0 | 0,674 |
| Negativkontrolle | pH 6,5 | 0,648 |
| | pH 7,0 | 0,327 |
| | pH 7,4 | 0,322 |
| Positivkontrolle | | 1,940 |

**Tabelle 3**

| (Photometrische Messung nach 20 Minuten) | | |
|---|---|---|
| Verbindung | initialer pH | OD (405-490 nm) |
| | pH 6,0 | 1,681 |
| CAM (800 ng/ml) | pH 6,5 | 1,708 |
| | pH 7,0 | 1,791 |
| | pH 7,4 | 2,217 |
| | pH 6,0 | 0,206 |
| 136 + 58a | pH 6,5 | 0,745 |
| (5 mM/10 mM) | pH 7, 0 | 2,036 |
| | pH 7,4 | 0,793 |
| | pH 6,0 | 0,750 |
| 136 + 121 | pH 6,5 | 0,486 |
| (5 mM/5 mM) | pH 7,0 | 2,564 |
| | pH 7,4 | 0,970 |
| | pH 6,0 | 0,419 |
| 136 + 132 | pH 6,5 | 0,328 |
| (5 mM/5 mM) | pH 7,0 | 0,802 |
| 1. Versuch | pH 7,4 | 0,953 |
| | pH 6,0 | 0,640 |
| 136 + 132 | pH 6,5 | 0,274 |
| (5 mM/5 mM) | pH 7,0 | 1,160 |
| 2. Versuch | pH 7,4 | 1,124 |
| | pH 6,0 | 0,211 |
| 136 + 188 | pH 6,5 | 1,728 |
| (5 mM/10 mM) | pH 7,0 | 1,426 |
| | pH 7,4 | 0,804 |
| | pH 6,0 | 0,577 |
| 58a + 121 | pH 6,5 | 0,480 |
| (10 mM/5 mM) | pH 7,0 | 2,227 |
| | pH 7,4 | 1,238 |
| | pH 6,0 | 0,152 |
| 58a + 132 | pH 6,5 | 0,241 |
| (10 mM/5 mM) | pH 7,0 | 0,417 |
| | pH 7,4 | 1,082 |
| | pH 6,0 | 0,251 |
| 58a + 188 | pH 6,5 | 1,698 |
| (10 mM/5 mM) | pH 7,0 | 2,939 |
| | pH 7,4 | 0,915 |
| | pH 6,0 | 0,407 |
| 132 | pH 6,5 | 0,371 |
| (5 mM) | pH 7,0 | 0,422 |
| | pH 7,4 | 0,916 |
| | pH 6,0 | 0,883 |
| Negativkontrolle | pH 6,5 | 0,794 |
| | pH 7,0 | 0,330 |
| | pH 7,4 | 0,579 |
| Positivkontrolle | | 1,219 |

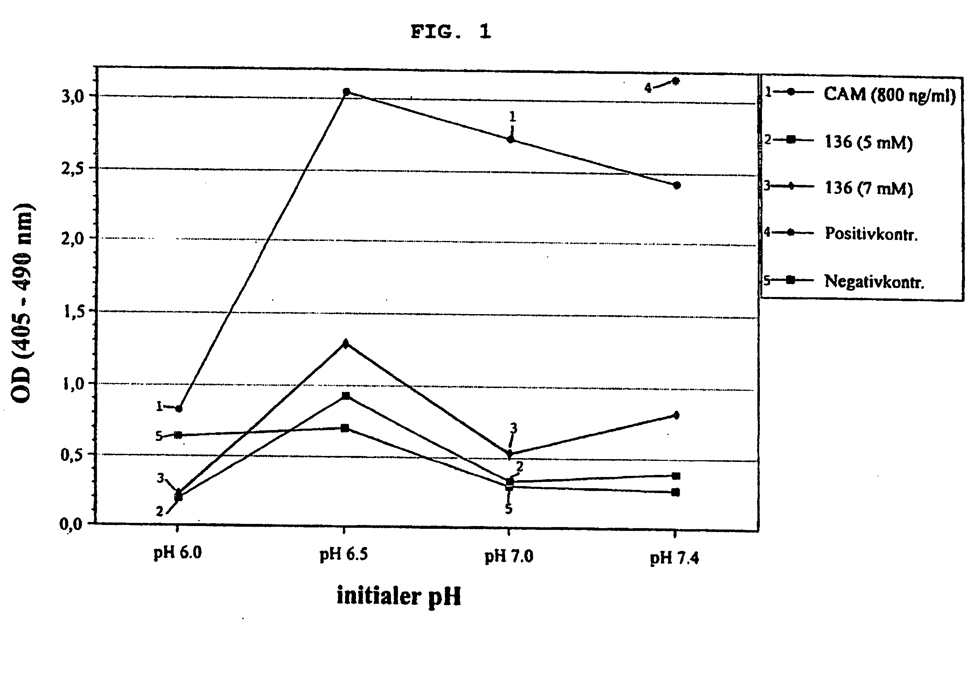

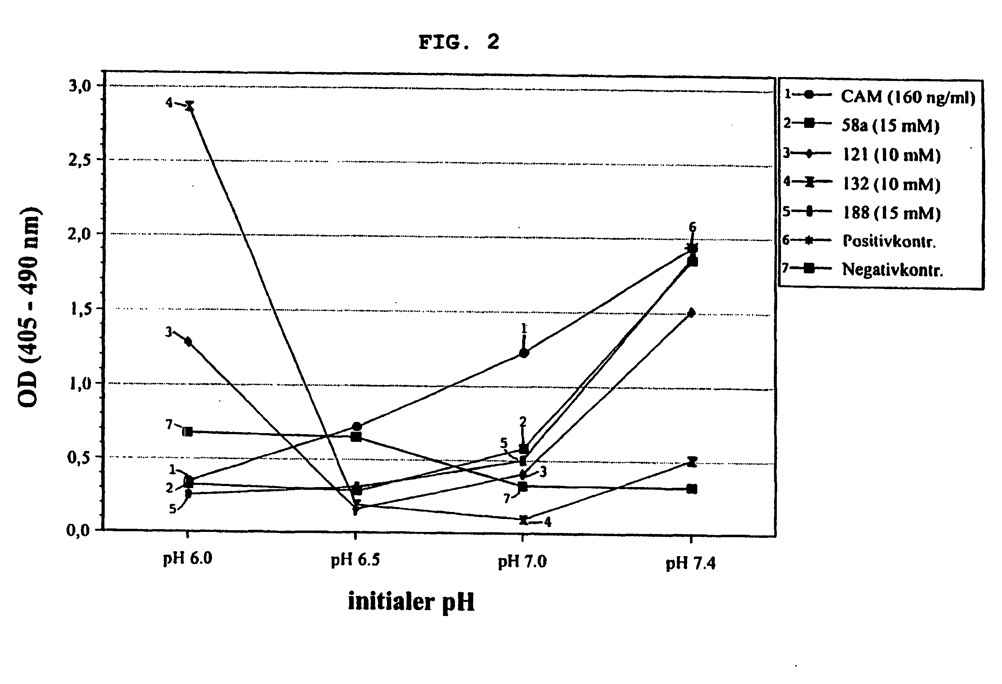

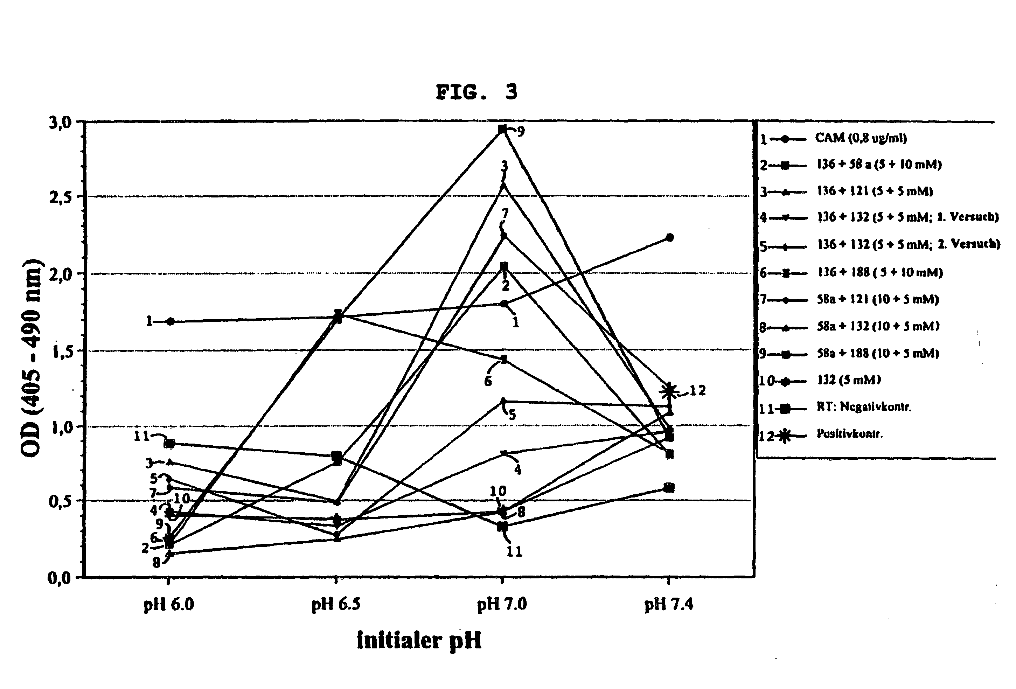

Die Ergebnisse der Versuche sind in den Abbildungen 1 bis 3 zusammengefaßt, die den Tabellen 1 bis 3 entsprechen. In den Abbildungen wird in einem ELISA (Apoptose-KIT) mit RT112 in Abhängigkeit vom pH-Wert der Zelltod aufgezeichnet. Das heißt, die Angabe auf der Y-Achse der Abbildungen ist ein Maß für den Zelltod, auf der X-Achse ist der pH-Wert der Zellen angegeben. Abbildung 1 und Abbildung 2 zeigen Versuche, bei denen Einzelverbindungen eingesetzt wurden. Abbildung 3 zeigt die synergistische Wirkung der erfindungsgemäßen Zusammensetzungen gegenüber den Einzelverbindungen. Es fällt auf, daß bei einem pH-Wert zwischen 6,5 und 7,0 die erfindungsgemäßen Zusammensetzungen einen Zelltod hervorrufen, der bevorzugt in der Nähe oder über dem CAM-Wert liegt, was praktisch einer vollständigen Zerstörung des Tumorgewebes entspricht.

Die in den Versuchen verwendete Apoptose auslösende Substanz CAM ist Camptothecin (CAM-Wert). In den Versuchen bedeutet Positivkontrolle, daß anstelle der durch die Substanzen induzierten apoptotischen Krebszellen ein Histon-DNA-Komplex mit bekannter Nachweisempfindlichkeit zur Detektion eingesetzt wird, das heißt ein künstliches Apoptose-Produkt wird vorgegeben. In den Versuchen bedeutet Negativkontrolle, daß die gleiche Nachweisprozedur durchgeführt wird, jedoch ohne Zugabe von Substanzen bzw. Substanzgemischen.

Aus den Vergleichsversuchen wird offensichtlich, daß bestimmte Gemische der vorstehend genannten Benzoesäurederivate eine synergistische Wirkung zeigen. Das Gemisch aus 2,4-Diacetoxybenzoesäure und 2-Hydroxy-4-aminobenzoesäure zeigt keine synergistische Wirkung, und die Fähigkeit des Gemischs, Tumorzellen abzutöten, ist nicht höher, als die von Einzelverbindungen, im Beispiel von 2,4-Diacetoxybenzoesäure.

Die erfindungsgemäßen Zusammensetzungen können auf an sich bekannte Art und Weise zu Arzneimitteln für Säuger, bevorzugt Menschen, formuliert werden. In den Arzneimitteln liegen die erfindungsgemäßen Zusammensetzungen im Gemisch mit einem pharmazeutischen organischen oder anorganischen Träger, der für enterale oder parenterale Verabreichungen geeignet ist, vor. Die orale Verabreichung der erfindungsgemäßen Zusammensetzungen über Tabletten, Kapseln, Pulver oder in flüssiger Form, wie als Suspensionen, in Lösung, als Emulsion oder als Sirup ist besonders bevorzugt.

Bei der Formulierung als Tabletten werden übliche Arzneimittelträger wie Natriumcitrat, Lactose, microkristalline Cellulose und Stärke, Schmiermittel wie wasserfreie Kieselsäure, hydriertes Castoröl, Magnesiumstearat, Natriumlaurylsulfat und Talk, sowie Bindemittel wie Stärkepaste, Glucose, Lactose, Gummi-Arabicum, Mannit, Magnesiumtrisilicat und Talk verwendet. Wenn die erfindungsgemäßen Zusammensetzungen über Flüssigkeiten verabreicht werden sollen, können übliche flüssige Träger verwendet werden.

Bevorzugt ist ebenfalls eine Formulierung für Injektionen und Infusionen, wie es auf dem Fachgebiet bekannt und in einschlägigen Standardwerken beschrieben ist.

Die erfindungsgemäßen Zusammensetzungen können ebenfalls als auf an sich bekannte Art und Weise als Depotformulierungen oder zu Arzneimitteln mit verzögerter oder hinhaltender Freisetzung formuliert werden.

Die Dosierungsform der erfindungsgemäßen Zusammensetzungen hängt von der speziellen Zusammensetzung und weiteren Faktoren ab und kann von einem Fachmann aufgrund des Zustands des zu behandelnden Patienten, der Schwere und Art der zu behandelnden Krankheit, möglicher Nebenwirkungen der verabreichten Substanzgemische, usw., bestimmt werden.

Die Dosierung der erfindungsgemäßen Zusammensetzungen kann von einem Fachmann in Abhängigkeit der speziellen Erkrankung, dem Patienten und sonstigen Umständen bestimmt werden und beträgt z.B. 50 mg/kg Körpergewicht bis 300 mg/kg Körpergewicht, bevorzugt 100 mg/kg Körpergewicht bis 200 mg/kg Körpergewicht der erfindungsgemäßen Zusammensetzung pro Tag.

Es ist für den Fachmann selbstverständlich, daß die Verbindungen der erfindungsgemäßen Zusammensetzungen zusammen verabreicht werden können oder nacheinander in derart kurzen Zeitabständen verabreicht werden können, daß sie ihre synergistische Wirkung noch aufweisen. Erfindungsgemäß ist sowohl die gleichzeitige Verabreichung eines geeignet formulierten Substanzgemischs umfaßt als auch das zeitversetzte oder gleichzeitige Verabreichen der geeignet formulierten einzelnen Bestandteile der erfindungsgemäßen Zusammensetzungen, sofern die Zeitabstände zwischen der Verabreichung der einzelnen Bestandteile nicht so groß sind, daß die synergistische Wirkung verlorengeht.

Der zeitliche Abstand zwischen der Verabreichung der einzeln formulierten Bestandteile beträgt im allgemeinen nicht mehr als 24 Stunden, bevorzugt nicht mehr als eine Stunde. Besonders bevorzugt werden die jeweiligen Formulierungen gleichzeitig oder unmittelbar nacheinander verabreicht.

Erfindungsgemäß kann das Arzneimittel daher als Arzneimittelkit vorliegen, bei dem ein Hinweis, beispielsweise auf dem Beipackzettel eines Benzoesäure-haltigen Präparates, vorhanden ist, daß dieses Benzoesäure-haltige Präparat gleichzeitig oder zeitlich versetzt mit einem anderen Benzoesäure-haltigen Präparat verabreicht werden soll. Ein derartiges Arzneimittelkit umfaßt auch zwei Arzneimittel, die jeweils in einer separaten Packung vorliegen, wobei auf dem Beipackzettel zumindest einer der Packungen auf die gleichzeitige oder zeitlich versetzte Verabreichung mit dem anderen Arzneimittel hingewiesen wird.

Das folgende galenische Beispiel erläutert die Erfindung und ist nicht einschränkend.

### Beispiel

Es wird eine Lösung aus 2-Hydroxy-4-aminobenzoesäure in destilliertem Wasser hergestellt, wobei die Konzentration der 2-Hydroxy-4-aminobenzoesäure so gewählt wird, daß die hergestellte Lösung isotonisch (34 mg/ml 2-Hydroxy-4-aminobenzoesäure) bis leicht hypertonisch (48 mg/ml 2-Hydroxy-4-aminobenzoesäure), steril und pyrogenfrei ist. Parallel dazu wird eine Lösung aus 2-Acetoxybenzoesäure, die unter dem Handelsnamen Aspisol erhältlich ist, bereitgestellt. Beide Lösungen werden nacheinander an einen Patienten intravenös verabreicht, wobei die Verabreichung der 2-Hydroxy-4-aminobenzoesäure in einer Dosierung von 300 mg/kg Körpergewicht und die Verabreichung der 2-Acetoxybenzoesäure in einer Dosierung von 50 mg/kg Körpergewicht erfolgt.

## Patentansprüche

1. Zusammensetzung, enthaltend ein Gemisch aus jeweils
2,6-Dihydroxy- und 2-Hydroxy-4-aminobenzoesäure,
2,6-Dihydroxy- und 2-Acetoxybenzoesäure (Acetylsalicylsäure),
2,6-Dihydroxy benzoe- und Salicylsäure,
2,6-Dihydroxy- und 2,4-Diacetoxybenzoesäure,
2,6-Dihydroxy- und 2,4-Dimethoxybenzoesäure,
2-Hydroxy-4-amino benzoe- und Salicylsäure,
2-Hydroxy-4-amino- und 2-Acetoxybenzoesäure (Acetylsalicylsäure),
2-Hydroxy-4-amino- und 2,4-Dimethoxybenzoesäure,
2,4-Dimethoxy- und 2-Acetoxybenzoesäure,
2,4-Dimethoxy benzoe- und Salicylsäure,
2,4,6-Trihydroxy- und 2,4-Dimethoxybenzoesäure,
2,4,6-Trihydroxy- und 2,6-Dihydroxybenzoesäure,
2,4,6-Trimethoxy- und 2,6-Dihydroxybenzoesäure,
2,4,6-Trimethoxy- und 2-Hydroxy-4-aminobenzoesäure,
2,4,6-Trimethoxy- und 2,4,6-Trihydroxybenzoesäure,
2,4,6-Trimethoxy- und 2,4-Dimethoxybenzoesäure,
2-Hydroxy-4-aminobenzoesäure und 5-(2,4-Difluorophenyl)-salicylsäure,
2-Acetoxybenzoesäure und 5-(2,4-Difluorophenyl)salicylsäure, Salicylsäure und 5-(2,4-Difluorophenyl)salicylsäure,
2-Acetoxybenzoesäure und α-Cyano-3-hydroxyzimtsäure, Salicylsäure und α-Cyano-3-hydroxyzimtsäure,
5-(2,4-Difluorophenyl)salicylsäure und α-Cyano-3-hydroxyzimtsäure,
2-Hydroxy-4-aminobenzoesäure und α-Cyano-3-hydroxyzimtsäure,
2-Hydroxy-4-aminobenzoesäure und 2-Acetoxybenzoesäure und 5-(2,4-Difluorophenyl)salicylsäure,
2-Hydroxy-4-aminobenzoesäure und Salicylsäure und 5-(2,4-Difluorophenyl)salicylsäure,
2-Hydroxy-4-aminobenzoesäure und 2-Acetoxybenzoesäure und α-Cyano-3-hydroxyzimtsäure,
2-Hydroxy-4-aminobenzoesäure und Salicylsäure und α-Cyano-3-hydroxyzimtsäure,
2-Hydroxy-4-aminobenzoesäure und 5-(2,4-Difluorophenyl)-salicylsäure und α-Cyano-3-hydroxyzimtsäure,
2-Acetoxybenzoesäure und 5-(2,4-Difluorophenyl) salicylsäure und α-Cyano-3-hydroxyzimtsäure oder
Salicylsäure und 5-(2,4-Difluorophenyl)salicylsäure und α-Cyano-3-hydroxyzimtsäure.

2. Zusammensetzung nach Anspruch 1 enthaltend 2-Hydroxy-4-aminobenzoesäure im Gemisch mit 2,4-Dimethoxybenzoesäure.

3. Zusammensetzung nach Anspruch 1 enthaltend 2,6-Dihydroxybenzoesäure im Gemisch mit Acetylsalicylsäure.

4. Zusammensetzung nach Anspruch 1 enthaltend 2-Hydroxy-4-aminobenzoesäure im Gemisch mit Acetylsalicylsäure.

5. Zusammensetzung nach Anspruch 1 enthaltend 2-Hydroxy-4-aminobenzoesäure im Gemisch mit 2,6-Dihydroxybenzoesäure.

6. Zusammensetzung nach Anspruch 1 enthaltend 2,6-Dihydroxybenzoesäure im Gemisch mit 2,4-Dimethoxybenzoesäure.

7. Zusammensetzung nach einem der Ansprüche 1 bis 6 zur Verwendung als Arzneimittel.

8. Zusammensetzung nach einem der Ansprüche 1 bis 6 zur Verwendung als Anti-Tumormittel.

9. Arzneimittel enthaltend eine Zusammensetzung nach einem der Ansprüche 1 bis 6 und einen pharmazeutisch verträglichen inerten Arzneimittelträger.

10. Arzneimittel nach Anspruch 9, **dadurch gekennzeichnet, daß** es die jeweiligen Wirkstoffe im Verhältnis 1:9 bis 9:1, vorzugsweise im Verhältnis 2:1 bis 1:2, und besonders bevorzugt im Verhältnis 1:1 aufweist.

11. Arzneimittel nach Anspruch 9 oder 10, **dadurch gekennzeichnet, daß** es zusätzlich Glucose enthält.

12. Verwendung einer Zusammensetzung nach einem der Ansprüche 1 bis 6 zur Herstellung eines Arzneimittels zur Behandlung von Krebserkrankungen.

## Claims

1. Composition comprising a mixture of
2,6-dihydroxy- and 2-hydroxy-4-aminobenzoic acid,
2,6-dihydroxy- and 2-acetoxybenzoic acid (acetylsalicylic acid),
2,6-dihydroxybenzoic acid and salicylic acid,
2,6-dihydroxy- and 2,4-diacetoxybenzoic acid,
2,6-dihydroxy- and 2,4-dimethoxybenzoic acid,
2-hydroxy-4-aminobenzoic acid and salicylic acid
2-hydroxy-4-amino- and 2-acetoxybenzoic acid (acetylsalicylic acid),
2-hydroxy-4-amino- and 2,4-dimethoxybenzoic acid,
2,4-dimethoxy- and 2-acetoxybenzoic acid,
2,4-dimethoxybenzoic acid and salicylic acid,
2,4,6-trihydroxy- and 2,4-dimethoxybenzoic acid,
2,4,6-trihydroxy- and 2,6-dihydroxybenzoic acid,
2,4,6-trimethoxy- and 2,6-dihydroxybenzoic acid,
2,4,6-trimethoxy- and 2-hydroxy-4-aminobenzoic acid,
2,4,6-trimethoxy- and 2,4,6-trihydroxybenzoic acid,
2,4,6-trimethoxy- and 2,4-dimethoxybenzoic acid,
2-hydroxy-4-aminobenzoic acid and 5-(2,4-difluorophenyl)-salicylic acid,
2-acetoxybenzoic acid and 5-(2,4-difluorophenyl)salicylic acid,
salicylic acid and 5-(2,4-difluorophenyl)salicylic acid,
2-acetoxybenzoic acid and α-cyano-3-hydroxycinnamic acid, salicylic acid and α-cyano-3-hydroxycinnamic acid,
5-(2,4-difluorophenyl)salicylic acid and α-cyano-3-hydroxycinnamic acid,
2-hydroxy-4-aminobenzoic acid and α-cyano-3-hydroxycinnamic acid,
2-hydroxy-4-aminobenzoic acid and 2-acetoxybenzoic acid and 5-(2,4-difluorophenyl)salicylic acid,
2-hydroxy-4-aminobenzoic acid and salicylic acid and 5-(2,4-difluorophenyl)salicylic acid,
2-hydroxy-4-aminobenzoic acid and 2-acetoxybenzoic acid and α-cyano-3-hydroxycinnamic acid,
2-hydroxy-4-aminobenzoic acid and salicylic acid and α-cyano-3-hydroxycinnamic acid,
2-hydroxy-4-aminobenzoic acid and 5-(2,4-difluorophenyl)-salicylic acid and α-cyano-3-hydroxycinnamic acid,
2-acetoxybenzoic acid and 5-(2,4-difluorophenyl)salicylic acid and α-cyano-3-hydroxycinnamic acid or
salicylic acid and 5-(2,4-difluorophenyl)salicylic acid and α-cyano-3-hydroxycinnamic acid.

2. The composition as claimed in claim 1 comprising 2-hydroxy-4-aminobenzoic acid as a mixture with 2,4-dimethoxybenzoic acid.

3. The composition as claimed in claim 1 comprising 2,6-dihydroxybenzoic acid as a mixture with acetylsalicylic acid.

4. The composition as claimed in claim 1 comprising 2-hydroxy-4-aminobenzoic acid as a mixture with acetylsalicylic acid.

5. The composition as claimed in claim 1 comprising 2-hydroxy-4-aminobenzoic acid as a mixture with 2,6-dihydroxybenzoic acid.

6. The composition as claimed in claim 1 comprising 2,6-dihydroxybenzoic acid as a mixture with 2,4-dimethoxybenzoic acid.

7. The composition as claimed in one of claims 1 to 6 for use as a medicament.

8. The composition as claimed in one of claims 1 to 6 for use as an antitumor agent.

9. A medicament comprising a composition as claimed in one of claims 1 to 6 and a pharmaceutically tolerable inert pharmaceutical excipient.

10. The medicament as claimed in claim 9, **characterized in that** it comprises the active agents in a ratio of 1:9 to 9:1, preferably in a ratio of 2:1 to 1:2 and most preferably in a ratio of 1:1.

11. The medicament as claimed in claim 9 or 10, **characterized in that** it additionally comprises glucose.

12. The use of a composition as claimed in one of claims 1 to 6 for the preparation of a medicament for the treatment of carcinomatous disorders.

## Revendications

1. Composition, contenant un mélange composé à chaque fois
d'acides 2,6-dihydroxy- et 2-hydroxy-4-aminobenzoïque,
d'acides 2,6-dihydroxy- et 2-acétoxybenzoïque (acide acétylsalicylique), benzoïque
d'acides 2,6-dihydroxy benzoïque- et salicylique,
d'acides 2,6-dihydroxy- et 2,4-diacétoxybenzoïque,
d'acides 2,6-dihydroxy- et 2,4-diméthoxybenzoïque,
d'acides 2-hydroxy-4-amino benzoïque- et salicylique,
d'acides 2-hydroxy-4-amino- et 2-acétoxybenzoïque (acide acétylsalicylique),
d'acides 2-hydroxy-4-amino- et 2,4-diméthoxybenzoïque,
d'acides 2,4-diméthoxy- et 2-acétoxybenzoïque,
d'acides 2,4-diméthoxy benzoïque- et salicylique,
d'acides 2,4,6-trihydroxy- et 2,4-diméthoxybenzoïque,
d'acide 2,4,6-trihydroxy- et 2,6-dihydroxybenzoïque,
d'acides 2,4,6-triméthoxy- et 2,6-dihydroxybenzoïque,
d'acides 2,4,6-triméthoxy- et 2-hydroxy-4-aminobenzoïque,
d'acides 2,4,6-triméthoxy- et 2,4,6-trihydroxybenzoïque,
d'acides 2,4,6-triméthoxy- et 2,4-diméthoxybenzoïque,
d'acide 2-hydroxy-4-aminobenzoïque et d'acide 5-(2,4-difluorophényl)-salicylique,
d'acide 2-acétoxybenzoïque et d'acide 5-(2,4-difluorophényl)salicylique,
d'acide salicylique et d'acide 5-(2,4-difluorophényl)salicylique,
d'acide 2-acétoxybenzoïque et d'acide α-cyano-3-hydroxycinnamique,
d'acide salicylique et d'acide α-cyano-3-hydroxycinnamique, acide 5-(2,4-difluorophényl)salicylique et d'acide α-cyano-3-hydroxycinnamique,
d'acide 2-hydroxy-4-aminobenzoïque et d'acide α-cyano-3-hydroxycinnamique,
d'acide 2-hydroxy-4-aminobenzoïque et d'acide 2-acétoxybenzoïque et d'acide 5-(2,4-difluorophényl)salicylique,
d'acide 2-hydroxy-4-aminobenzoïque et d'acide salicylique et acide 5-(2,4-difluorophényl)-salicylique,
d'acide 2-hydroxy-4-aminobenzoïque et d'acide 2-acétoxybenzoïque et d'acide α-cyano-3-hydroxycinnamique,
d'acide 2-hydroxy-4-aminobenzoïque et d'acide salicylique et acide α-cyano-3-hydroxycinnamique,
d'acide 2-hydroxy-4-aminobenzoïque et d'acide 5-(2,4-difluorophényl)salicylique et d'acide b-cyano-3-hydroxycinnamique,
d'acide 2-acétoxybenzoïque et d'acide 5-(2,4-difluorophényl)-salicylique et d'acide α-cyano-3-hydroxycinnamique ou
d'acide salicylique et d'acide 5-(2,4-difluorophényl)salicylique et d'acide α-cyano-3-hydroxycinnamique.

2. Composition selon la revendication 1, contenant de l'acide 2-hydroxy-4-aminobenzoïque mélangé avec de l'acide 2,4-diméthoxybenzoïque.

3. Composition selon la revendication 1, contenant de l'acide 2,6-dihydroxybenzoïque mélangé avec de l'acide acétylsalicylique.

4. Composition selon la revendication 1, contenant de l'acide 2-hydroxy-4-aminobenzoïque mélangé avec de l'acide acétylsalicylique.

5. Composition selon la revendication 1, contenant de l'acide 2-hydroxy-4-aminobenzoïque mélangé avec de l'acide 2,6-dihydroxybenzoïque.

6. Composition selon la revendication 1, contenant de l'acide 2,6-dihydroxybenzoïque mélangé avec de l'acide 2,4-diméthoxybenzoïque.

7. Composition selon l'une quelconque des revendications 1 à 6 pour une utilisation en tant que médicament.

8. Composition selon l'une quelconque des revendications 1 à 6 pour une utilisation en tant qu'agent anti-tumoral.

9. Médicament contenant une composition selon l'une quelconque des revendications 1 à 6 et véhicule pour médicaments inerte, acceptable sur le plan pharmaceutique.

10. Médicament selon la revendication 9, **caractérisé en ce qu'**il contient chacun des principes actifs dans un rapport allant de 1:9 à 9:1, de préférence dans un rapport allant de 2:1 à 1:2, et de manière plus particulièrement préférée dans un rapport de 1:1.

11. Médicament selon la revendication 9 ou 10, **caractérisé en ce qu'**il contient en plus du glucose.

12. Utilisation d'une composition selon l'une quelconque des revendications 1 à 6 pour la préparation d'une médicament en vue du traitement de maladies cancéreuses.
